# EUROPEAN PATENT SPECIFICATION

(11) **EP 0 563 060 B1**
(45) Date of publication and mention of the grant of the patent: **06.05.1999**
(21) Application number: 91920247.3
(22) Date of filing: 01.10.1991
(51) Int. Cl.: A61K 45/05, A61K 38/21, C07K 14/00, A61K 38/00

(54) **Use of IL-4 and TNF for the preparation of a medicament for the treatment of tumour cells**
Die Verwendung von IL-4 und TNF zur Herstellung eines Medikaments zur Behandlung von Krebszellen
Utilisation de l'IL-4 et TNF pour l'obtention d'un médicament destiné au traitement de cellules cancérieuses

(30) Priority: 01.10.1990 US 590977
(43) Date of publication of application: 06.10.1993
(73) Proprietor: RESEARCH DEVELOPMENT FOUNDATION, Carson City Nevada 89703 (US)
(72) Inventor: AGGARWAL, Bharat, Houston, TX 77005 (US)
(74) Representative: Wilkinson, Stephen John
(86) International application number: US9107234
(87) International publication number: WO9205805

(56) References cited:
- US-A- 4 650 674
- US-A- 4 770 995
- US-A- 4 962 091
- IMMUNOLOGY vol. 70, no. 4, August 1990, GREAT BRITAIN pages 498 - 503 PHILLIPS ET AL. 'Priming the macrophage respiratory burst with IL-4: enhancement with tNF- alpha but inhibition by IFN-gamma'
- Blood, Volume 75, No. 5, issued 01 March 1990, TAYLOR et al., "Effects of interleukin-4 on the vitro growth of human lymphoid and plasma cell neoplasms", pages 1114-1118, see page 1114 under "Drug sensitivity testing".
- The Journal of Immunology, Volume 144, No. 8, issued 15 April 1990, SUDA et al., "Growth-promoting activity of IL-1x, IL-6, and tumor necrosis factor-x in combination with IL02, IL-4, or IL-7 on nurine thymocytes. Differential effects on CD4/CD8 subsets and on CD3+/CD3- double- negative thymocytes:, pages 3039-3045, see Tables I and II.
- The Journal of Immunology, Volume 144, No. 12, issued 15 June 1990, KATZ et al., "Enhanced synthesis of factor B induced by tumor necrosis factor in human skin fibroblasts is decreased by IL-4", pages 4675-4680, see Figs. 1-6.
- Cellular Immunology, Volume 128, issued July 1990, SWISHER et al., "TNF-x and IFM-X reverse IL-4 inhibition of lymphokine activated killer cell function", pages 450-461, see Tables 4-5 and Figs. 1-3.
- The Journal of Immunology, Volume 145, No. 3, issued 01 August 1990, THORNHILL et al., "IL-4 regulates endothelial cell activation by IL-1, tumor necrosis factor, or IFN-X", pages 865-872, see Tables I-VII and Figs. 3-5.
- International Journal of Cancer, Volume 4 (Supplement), issued November 1989, FORNI et al., "Lymphokine-activated tumor inhibition" combining activity of a synthetic nonapeptide from interleukin-1, interleukin-2, interleukin-4, and interferon-X injected around tumor-draining lymph nodes", pages 62-65, see Abstract; fig. 2.

## Description

### Field of the Invention

This invention relates to lymphokines. In particular, it relates to the synergistic effects of TNF and IL-4 on inhibition of tumor cell growth and cytotoxicity.

### Background of the Invention

A cytokine is any kind of molecule produced by one cell that affects it or other cells. There is a wide variety of cytokines which, as a group, exert a variety of effects on both normal and abnormal cells. The effect on a particular cell of a specific cytokine is dependent both on the specific cytokine and the type of target cell.

Tumor necrosis factor (TNF) is a cytokine which is primarily a product of activated monocytes and is highly pleiotropic in nature (Ayier, et al. (1988) Tumor Necrosis Factors, CRC Handbook on Cytolytic Lymphocytes and Complement: Effectors of the Immune System (E.R. Podack, ed.), CRC Press Inc., Boca Raton, Fla., p. lO5; Goeddel, et al. (1987) Tumor Necrosis Factors: Gene Structure and Biological Activities. In Cold Spring Harbor Symposia on Quantitative Biology, L1:597). It inhibits the growth of a wide variety of tumor cells in culture and stimulates the growth of fibroblasts, B-cells and thymocytes. The cytotoxic effects of TNF against tumor cells are potentiated by interferons (Lee, et al., (1984) J. Immunol. 133:1003; Sugarman, et al. (1985) Science 230:943; Aggarwal, et al. (1985) Nature 318:665; Vilcek, et al. (1986) Interaction Between Tumor Necrosis Factor and Interferons, in The Biology of the Interferon System. Edited by H. Schellekenes and W.E. Stewart. Elsevier Science Publishers, Amsterdam., p. 249; Fransen, et al. (1986) Eur. J. Cancer Clin. Oncol. 22:419-426; Campbell, et al. (1988) J. Immunol. 141:2325-2329; Schiller, et al. (1987) Cancer Res. 47:2809-2813). The enhancement of the anti-proliferative effects of TNF by interferons accompanies the upregulation of TNF receptors (Aggarwal, et al. (1985) Nature 318:665; Fransen, et al. (1986) Eur. J. Cancer Clin. Oncol. 22:419-426). The upregulation of TNF receptors by interferons, however, is not a primary mechanism for their synergistic cytotoxic response (Tsujimoto, et al. (1986) J. Immunol. 137:2272-2276; Aggarwal and Eessalu (1987) J. Biol. Chem. 262:10000-10007).

The synergistic effects of interferon-g and TNF on tumor growth regulation were disclosed in U.S. Patent No. 4,650,674.

Interleukin-4 (IL-4) is an immunomodulatory cytokine produced by activated T-lymphocytes. One of a number of immune system modulators, it was described originally as a B-cell growth factor because of its ability to co-stimulate B-lymphocyte proliferation (Howard, et al. J. Exp. Med. 155:914 (1982). IL-4 also affects a wide variety of cells including T-cells, mast cells, macrophages, and hematopoietic progenitor cells (Paul, et al. (1987) Ann. Rev. Immunol. 5:429; Yokota, et al. (1988) Immunol. Rev. 102:137; Crabstein et al., (1986) J. Exp. Med. 163:1405). IL-4 enhances the growth of several types of cells including T-cells and mast cells (Fernandez-Botran, et al. (1986) Proc. Natl. Acad. Sci. USA 83:9689; Spits, et al. (1987) J. Immunol. 139:1142; Zlotnik, et al. (1987) Proc. Natl. Acad. Sci. USA 84:3856; Mosmann, et al. (1986) Proc. Natl. Acad. Sci. USA 83:5654), induces MHC class II antigen expression on B-cells and monocytes (TeVelde, et al. (1988) J. Immunol. 140:1548), augments antigen specific cytotoxic T-cells and enhances the macrophage mediated cytotoxicity (Spits, et al. (1988) J. Immunol. 141:29; Widmer, et al. (1987) J. Exp. Med. 166:1447; Crawford, et al. (1987) J. Immunol. 139:135). While IL-4 downregulates the production of several cytokines including IL-1a, IL-b, IFN-g and TNF-a, it also upregulates the production of IL-6 from B-cells (Essner, et al. (1989) J. Immunol. 142:3857; Lee, et al. (1990) J. of Leukocyte Biol. 47:475-479; Pelman, et al. (1989) J. Exp. Med. 170:1751). IL-2-induced LAK and NK cell activity is downmodulated by IL-4 (Brooks, et al. (1988) Clin. Exp. Immunol. 74:162; Nagler, et al. (1988) J. Immunol. 141:2349). Besides proliferative effects of IL-4, it has also recently been shown to inhibit the growth in vitro of human lymphoid and plasma cell neoplasms (Taylor, et al. (1990) Blood 75:5). Brief Description of the Invention

The present invention relates to the synergistic effects of IL-4 on the antiproliferative activity of TNF. IL-4 potentiates the cytotoxic effects of TNF against a variety of tumor cells, including but not limited to breast tumor cells, carcinoma cells, and lymphoma cells. These synergistic effects were dependent on the dose of TNF and of IL-4. The enhancement of TNF cytotoxicity by IL-4 is comparable with that reported previously for interferon gamma.

The present invention relates to the synergistic effects of a T-cell derived cytokine, Interleukin-4, on the cytotoxic effects of TNF in tumor cells. In one embodiment, the present invention provides the use of a cell-free composition comprising IL-4 and TNF in a unit activity proportion of from 0.1:1 to 200:1 for the manufacture of a medicament for the treatment of tumor cells. In another, there is provided the use of a cell-free composition comprising IL-4 and lymphotoxin in a unit activity proportion of from 0.1:1 to 200:1 for the manufacture of a medicament for the treatment of tumor cells. According to a preferred embodiment, the cell free composition comprising IL-4 and lymphotoxin also contains interferon-gamma.

The antiproliferative effects of TNF against a number of different cell lines are potentiated by IL-4. IL-4 alone had no growth inhibitory effects against these cell lines or, in some cases, was slightly growth stimulatory. The degree of enhancement of cytotoxic effects of TNF by IL-4 was comparable quantitatively with that of IFN-g; however, the mechanisms by which IL-4 and IFN-g potentiate the antiproliferative or cytotoxic effects of TNF and Lymphotoxin (Lt) are different.

Administration of a cell free composition suitable for the treatment of tumor cells comprising tumor necrosis factor and Interleukin-4 to a tumor bearing animal results in a decrease in the tumor growth. Preferably, the tumor necrosis factor is human tumor necrosis factor. The tumor necrosis factor may be obtained from any source. Such sources are known to those of skill in the art. Preferably, the tumor necrosis factor is recombinantly produced.

Preferably, the IL-4 is human IL-4. The IL-4 may be obtained from any source. Such sources are known to those of skill in the art. Most preferably, the IL-4 is recombinantly produced.

### Brief Description of the Drawings

Figure 1 demonstrates the effect of different concentrations of TNF and IL-4 (100 ng/ml) on the cell viability of a human breast tumor cell line (MDA-MB-330).
Figures 2A and 2B demonstrate the effect of different concentrations of TNF and IL-4 (100 ng/ml) on the cell viability of a human epithelial carcinoma cell line (A-431; Figure 2A) and human histiocytic lymphoma cell line (U-937; Figure 2B).
Figures 3A and 3B demonstrate the effect of different concentrations of IL-4 on the TNF-dependent cytotoxicity against A-431 (Figure 3A) and MDA-MB-330 (Figure 3B) cell lines.
Figure 4 demonstrates the comparison of the effect of IL-4 and IFN-g on the TNF dependent cytotoxicity against MDA-MB-330 cells.
Figure 5 demonstrates the comparison of the effect of IL-4 on TNF and Lt dependent cytotoxicity against MDA-MB-330 cells.

### Detailed Description of the Invention

The present invention is based on the discovery by the inventors that the antiproliferative activity of TNF on tumor cells is enhanced by the administration of IL-4 to the tumor cells concurrently with TNF. IL-4 potentiates the cytotoxic effects of TNF against a variety of tumor cells, including but not limited to, breast tumor cells (MDA-MD-330), vulvar carcinoma cells (A431), and histiocytic lymphoma (U-937) cells. This synergistic effect was dependent on the dose of TNF and of IL-4. IL-4 alone had minimal effects on these cell lines when administered alone. In fact, on some cell lines, IL-4 exhibited significant proliferative activity when administered alone. The enhancement of TNF cytotoxicity by IL-4 is comparable with that reported previously for interferon gamma (IFN-g).

A composition comprising a T-cell derived cytokine, Interleukin-4, and TNF has a greater cytotoxic effect on tumor cells than does TNF administered alone. In one embodiment, the composition of matter used for the manufacture of a medicament for the treatment of tumor cells comprises substantially purified TNF and IL-4. These cytokines may be derived from any source, including native or recombinantly produced proteins. This composition of matter may comprise substantially purified TNF, IL-4 and interferon-gamma (IFN-g).

The term "individual" is meant to include any animal, preferably a mammal, and most preferably a rodent, cat, dog, cow or human.

The term "Tumor Necrosis Factor" as used herein refers to a polypeptide other than lymphotoxin capable of preferential cytotoxic activity and having a region showing functional amino acid homology with the mature tumor necrosis factor amino acid sequence, a fragment thereof, or a derivative of such polypeptide or fragment. A detailed description of tumor necrosis factor useful in practicing the present invention is disclosed in US Patent No. 4,650,674.

Preferential cytotoxic activity is defined as the preferential destruction or growth inhibition of tumor cells when compared to normal cells under the same conditions. Preferential cytotoxic activity is detected by the effect of the polypeptide on tumor cells in vivo or in vitro as compared with normal cells or tissue. Cell lysis is generally the diagnostic indicia in vitro, while tumor necrosis is determined in in vivo experiments. However, cytotoxic activity may be manifest as cytostasis or antiproliferative activity. Suitable assay systems are well known. For example, the cell-lytic assay used to determine the specific activity of tumor necrosis factor described below is acceptable, as is the assay described in B. Aggarwal, et al. in "Thymic Hormones and Lymphokines," 1983, ed. A. Goldstein, Spring Symposium on Health Sciences, George Washington University Medical Center (the A549 cell line referred to in this literature is available from the ATCC as CCL185).

The specific activity of TNF is cast in terms of target cell lysis rather than cytostasis. One unit of tumor necrosis factor is defined as the amount required for 50 percent lysis of target cells. However, this is not meant to exclude other assays for measuring specific activity, e.g. methods based on target cell growth rate.

Native tumor necrosis factor from normal biological sources has a calculated molecular weight of about 17,000 on sodium dodecyl sulfate polyacrylamide gel electrophoresis (SDS-PAGE), an isoelectric point of about 5.3, and susceptibility to trypsin hydrolysis at multiple sites. Native tumor necrosis factor has been purified by reverse phase HPLC.

Generally a polypeptide defined as tumor necrosis factor will contain regions substantially homologous with the tumor necrosis factor protein described in U.S. Patent No. 4,650,674 or fragments thereof over a continuous block of about from 20 to 100 amino acid residue, in particular the blocks encompassed by residues 35-66 and 110-133.

A most significant factor in establishing the identity of a polypeptide as tumor necrosis factor is the ability of antisera which is capable of substantially neutralizing the cytolytic activity of mature tumor necrosis factor to also substantially neutralize the cytolytic activity of the polypeptide in question. However, it will be recognized that immunological identity and cytotoxic identity are not necessarily coextensive. A neutralizing antibody for tumor necrosis factor may not bind a candidate protein because the neutralizing antibody happens to not be directed to specifically bind a site on tumor necrosis factor that is critical to its cytotoxic activity. Instead, the antibody may bind an innocuous region and exert its neutralizing effect by steric hinderance. Therefore, a candidate protein mutated in this innocuous region might no longer bind the neutralizing antibody, but it would nonetheless be tumor necrosis factor in terms of substantial homology and biological activity.

It is important to observe that characteristics such as molecular weight, isoelectric point and the like for the native or wild type mature human tumor necrosis factor obtained from peripheral lymphocyte or established cell line cultures are descriptive only for the native species of tumor necrosis factor. Tumor necrosis factor as contemplated by the foregoing definition will include other species which will not exhibit all of the characteristics of native tumor necrosis factor. While tumor necrosis factor as defined herein includes native tumor necrosis factor, other related cytotoxic polypeptides will fall within the definition. For example, TNF derivatives like the insertion mutants, deletion mutants, or fusion proteins will bring tumor necrosis factor outside of the molecular weight established for native human tumor necrosis factor (Fusion proteins with mature tumor necrosis factor or pre TNF itself as well as insertion mutants will have a greater molecular weight than native, mature tumor necrosis factor, while deletion mutants of native, mature tumor necrosis factor will have a lower molecular weight.) Similarly, tumor necrosis factor may be engineered in order to reduce or eliminate susceptibility to hydrolysis by trypsin or other proteases. Finally, post-translational processing of human pre TNF in cell lines derived from nonprimate mammals may produce microheterogeneity in the amino terminal region so that valine will no longer be the amino terminal amino acid.

Note that the language "capable" of cytotoxic activity or in vivo tumor necrosis means that the term tumor necrosis factor includes polypeptides which can be converted, as by enzymatic hydrolysis, from an inactive state analogous to a zymogen to a polypeptide fragment which exhibits the desired biological activity. Typically, inactive precursors will be fusion proteins in which mature tumor necrosis factor is linked by a peptide bond at its carboxyl terminus to a human protein or fragment thereof. The sequence at this peptide bond or nearby is selected so as to be susceptible to proteolytic hydrolysis to release tumor necrosis factor, either in vivo or, as part of a manufacturing protocol, in vitro. The tumor necrosis factor that is so generated then will exhibit the definitionally-required cytotoxic activity.

While tumor necrosis factor ordinarily is meant to mean human tumor necrosis factor, tumor necrosis factor from sources such as murine, porcine, equine or bovine is included within the definition of tumor necrosis factor so long as it otherwise meets the standards described above for homologous regions and cytotoxic activity. TNF is not species specific, e.g., human TNF is active on mouse tumors. Therefore, TNF from one species can be used in therapy of another.

Tumor necrosis factor also includes multimeric forms. Derivatives of tumor necrosis factor are included within the scope of the term tumor necrosis factor. The regions of the tumor necrosis molecule within residues 35 to 66 and 110 to 133 inclusive show substantial homology (50 percent) with lymphotoxin. The hydrophobic carboxy-termini (tumor necrosis factor residues 150 to 157) of the two molecules also are significantly conserved. Since both proteins demonstrate cytotoxic activity or in vivo tumor necrosis, these regions are believed to be important in the shared activity of lymphotoxin and tumor necrosis factor. Therefore, in practicing the present invention, lymphotoxin may be substituted in the composition of the present invention in place of TNF.

The term "Interleukin-4" or "IL-4" as used herein refers to a polypeptide originally described as a factor produced by EL-4 thymoma cells following activation by TPA, which induced proliferation of murine B-cells activated by submitogenic doses of anti-Ig. IL-4 has also been termed B-cell Growth Factor (BOGF), B-cell Stimulatory Factor (BSF-1), T-cell Growth Factor II (TCGF-II) and Mast cell Growth Factor-II (MCFG-II) (Howard, et al. (1982) J. Exp. Med. 155:914-923; Yokota, et al. (1988) Immunol. Rev. 102:137. IL-4, produced by a variety of T-cell/lymphocyte populations, may be detected by immunoassays (ELISAs) and Northern blot analysis (Paliard, et al. 1988). Typically, IL-4 activity may be determined in a bioassay of the T-cell growth promoting activity such as that disclosed by Spits, et al. (1987) J. Immunol. 139:1142-1147. However, other methods for assaying the activity of and identifying IL-4 are known to those of skill in the art. For instance, a typical bioassay is performed by activating peripheral blood mononuclear cells with PHA for 72 hours at 37°C, washing the PHA-blasts and plating 5000 cells/0.1 ml/well in a 96-well plate. The IL-4 test samples are serially diluted and incubated with the cells for 72 hours at 37°C. Tritiated thymidine was added to the cells and the IL-4 activity was determined by measuring the tritiated thymidine incorporation into the cells. One unit of IL-4 is defined as the amount of cytokine giving half maximal proliferation.

The purification of naturally occurring IL-4 from serum containing culture supernatants has been described by Le, et al. (1988) J. Biol. Chem. 263:10817-10823. Briefly, after cation exchange chromatography on S-Sepharose, the IL-4 containing fractions eluted with a NaCl gradient from 0-0.5M in 50 mM HEPES, pH 7.0, were subjected to gel filtration on Sephadex G-100. The IL-4 fractions are further purified by Reversed phase HPLC, using a gradient of 20-70 percent (v/v) acetonitrite in 0.1 percent (v/v) trifluoroacetic acid.

These procedures result in recoveries of approximately 60 percent and a 3000-fold purification (specific activity: 2.6x10⁷ U/mg). Purification of IL-4 from serum free culture supernatants requires only one chromatography step on CM-Sepharose resulting in recoveries of 40 percent and a 100-fold purification (specific activity: 3x10⁷ U/mg).

Any polypeptide having IL-4 activity and having a region showing functional amino acid homology with the mature IL-4 amino acid sequence, a fragment thereof, or a derivative of such polypeptide or fragment will be useful in practicing the present invention.

The specific activity of IL-4 is cast in terms of cell proliferation, despite the fact that its use in the present invention is the potentiation of TNF cytotoxicity. However, this is not meant to exclude other assays for measuring specific activity, e.g. methods based on other biological effects on target cells.

The physicochemical properties of IL-4 have been described by Yokota, et al. (1988) Immunol. Rev. 102:137 and Le, et al. (1988) J. Biol. Chem. 263:10817-10823. Native IL-4 from normal biological sources has a calculated molecular weight of about 20,000-22,000 Da as determined by gel filtration, an isoelectric point of about 6.7 when the sugar groups have been removed and 10.53 with the sugar groups, and susceptibility to trypsin and chymotrypsin hydrolysis. The IL-4 protein sequence has 129 amino acids which yields a theoretical molecular weight of 15,000 daltons. This theoretical molecular weight is close to the molecular weights determined by SDS-PAGE of 15,000, 18,000, and 19,000. The discrepancy between the molecular weight determined by gel filtration and SDS-PAGE is accounted for by the carbohydrate moieties.

Recombinant IL-4 has a calculated molecular weight of about 22,000 Da determined by gel filtration and 15,000 by SDS-Page, an isoelectric point of about 10.53, and susceptibility to trypsin and chymotrypsin hydrolysis.

It is stable for more than 3 months at 4°C, more than 1 month at room temperature, and for about 10 minutes at 56°C. IL-4 is stable over a wide pH range (2-10). IL-4 is not species specific in that human IL-4 acts on murine cells, but murine IL-4 is species specific.

The degree of amino acid sequence homology which brings a polypeptide within the scope of the definition of IL-4 herein will vary depending upon whether the homology between the candidate protein and IL-4 falls within or without the IL-4 regions responsible for cytotoxic activity. Domains which are critical for cytotoxic activity should exhibit a high degree of homology in order to fall within the definition, while sequences not involved in maintaining IL-4 conformation or in effecting receptor binding may show comparatively low homology. In addition, critical domains may exhibit cytolytic activity and yet remain homologous as defined herein if residues containing functionally similar amino acid side chains are substituted. Functionally similar refers to dominant characteristics of the side chains such as basic, neutral or acid, or the presence or absence of steric bulk.

A most significant factor in establishing the identity of a polypeptide as IL-4 is the ability of antisera which is capable of substantially neutralizing the biological activity of IL-4 to also substantially neutralize the biological activity of the polypeptide in question. However, it will be recognized that immunological identity and cytotoxic identity are not necessarily coextensive. A neutralizing antibody for IL-4 may not bind a candidate protein because the neutralizing antibody happens to not be directed to specifically bind a site on IL-4 that is critical to its biological activity. Instead, the antibody may bind an innocuous region and exert its neutralizing effect by steric hinderance. Therefore, a candidate protein mutated in this innocuous region might no longer bind the neutralizing antibody, but it would nonetheless be IL-4 in terms of substantial homology and biological activity.

It is important to observe that characteristics such as molecular weight, isoelectric point and the like for the native or wild type mature IL-4 obtained from peripheral lymphocyte or established cell line cultures are descriptive only for the native species of IL-4. IL-4 as contemplated by the foregoing definition will include other species which will not exhibit all of the characteristics of native IL-4. While IL-4 as defined herein includes native IL-4, other related polypeptides will fall within the definition. For example, IL-4 derivatives like insertion mutants, deletion mutants, or fusion proteins will bring IL-4 outside of the molecular weight established for native IL-4 (fusion proteins with mature IL-4 as well as insertion mutants will have a greater molecular weight than native, mature IL-4, while deletion mutants of native IL-4 will have a lower molecular weight). Similarly, IL-4 may be engineered in order to reduce or eliminate susceptibility to hydrolysis by trypsin or other proteases.

The composition used in the present invention may be administered with any one or all of the polypeptides in an inactive form which can be converted, as by enzymatic hydrolysis, from an inactive state analogous to a zymogen to a polypeptide fragment which exhibits the desired biological activity. Typically, inactive precursors will be fusion proteins in which one or more of IL-4, TNF and or IFN-g is linked by a peptide bond at its carboxyl terminus to a human protein or fragment thereof or either directly or indirectly to another of the polypeptides useful in practicing the present invention. The sequence at this peptide bond or nearby is selected so as to be susceptible to proteolytic hydrolysis to release the IL-4, tumor necrosis factor, and IFN-g either in vivo or, as part of a manufacturing protocol, in vitro. The composition that is so generated then will exhibit the definitionally-required cytotoxic activity.

While IL-4 ordinarily is meant to mean human IL-4, IL-4 from other sources such as murine, porcine, equine or bovine is included within the definition of IL-4 so long as it otherwise meets the standards described above for homologous regions and cytotoxic activity.

Multimeric forms of IL-4 may also be used to practice the present invention.

Derivatives of IL-4 are included within the scope of the term IL-4. Derivatives include amino acid sequence mutants, glycosylation variants and covalent or aggregative conjugates with other chemical moieties. Covalent derivatives are prepared by linkage of functionalities to groups which are found in the IL-4 amino acid side chains or at the N- or C-termini by means known in the art.

As with TNF, mutant IL-4 derivatives include the predetermined, i.e. site specific, mutations of IL-4 or its fragments which are useful in practicing the invention. Mutant IL-4 is defined as a polypeptide otherwise falling within the homology definition for IL-4 set forth herein but which has an amino acid sequence different from that of IL-4 whether by way of deletion, substitution or insertion. Techniques for making substitution mutations at predetermined sites in DNA having a known sequence are well known, for example M13 primer mutagenesis.

Substantially homogeneous IL-4 or tumor necrosis factor means IL-4 or tumor necrosis factor which is substantially free of other proteins native to the source from which the native protein was isolated. This means that homogeneous IL-4 and/or tumor necrosis factor is substantially free of blood plasma proteins such as albumin, fibrinogen, serine proteases, a-globulins, non-tumor necrosis factor cytotoxic polypeptides such as lymphotoxin or interferons, or other proteins of the cell or organism which serve as the synthetic origin of the polypeptide, including whole cells and particulate cell debris. However, substantially pure polypeptides may include such substances as the stabilizers and excipients described below, predetermined amounts of proteins from the cell or organism that serve as the synthetic origin, proteins from other than the original polypeptide source cells or organisms, and synthetic polypeptides such as poly-L-lysine. Recombinant IL-4 or tumor necrosis factor which is expressed in an allogeneic, e.g. bacterial, host cell of course will be expressed completely free of gene source proteins.

IL-4 and tumor necrosis factor used in practicing the present invention are preferably synthesized in cultures of recombinant organisms by means known to those in the art.

The composition is prepared for administration by mixing tumor necrosis factor and IL-4 having the desired degree of purity with physiologically acceptable carriers, i.e. carriers which are nontoxic to recipients at the dosages and concentrations employed. Ordinarily, this will entail combining the IL-4 and TNF composition with buffers, antioxidants such as ascorbic acid, low molecular weight (less than about 10 residues) polypeptides, proteins, amino acids, carbohydrates including glucose or dextrins, chelating agents such as EDTA, and other stabilizers and excipients. The carrier should be formulated to stabilize the composition. The composition to be used for therapeutic administration must be sterile. This is readily accomplished by filtration through sterile filtration membranes. The composition ordinarily will be stored in lyophilized form.

In addition, since interferons act synergistically with IL-4 and tumor necrosis factor, alpha, beta or gamma interferon is desirably combined with tumor necrosis factor/IL-4 compositions or IL-4/tumor necrosis factor and lymphotoxin-containing compositions. TNF has a specific activity of about 100 U/ng. The specific activity of IL-4 and IFN-g is about 10 U/ng. A typical formulation comprises IL-4 and/or IFN, and tumor necrosis factor and/or lymphotoxin in a unit activity proportion of from 0.1:1 to 200:1, ordinarily 10 to 1, and may contain lymphotoxin in a place of a proportion or all of the tumor necrosis factor. These proportions, of course, are subject to modification as required by therapeutic experience.

IL-4/TNF compositions are administered to tumor-bearing animals. The route of administration is in accord with known methods, e.g. the intravenous, intraperitoneal, intramuscular, intralesional infusion or injection of sterile IL-4/TNF solutions, or by timed release systems as noted below. IL-4/TNF can be administered intralesionally, i.e. by direct injection into solid tumors. In the case of disseminated tumors such as leukemia, administration is preferably intravenous or into the lymphatic system. Tumors of the abdominal organs such as ovarian cancer are advantageously treated by intraperitoneal infusion using peritoneal dialysis hardware and peritoneal-compatible solutions. Ordinarily, however, IL-4/TNF is administered continuously by infusion although bolus injection is acceptable.

IL-4/TNF desirably is administered from an implantable timed-release article. Examples of suitable systems for proteins having the molecular weight of tumor necrosis factor dimers or trimers include copolymers of L-glutamic acid and gamma ethyl-L-glutamate (U. Sidman, et al., 1983, "Biopolymers" 22(1): 547-556), poly (2-hydroxyethyl-methacrylate) (R. Langer, et al., 1981, "J. Biomed. Matter. Res." 15: 167-277 and R. Langer, 1982, "Chem. Tech." 12: 98-105) or ethylene vinyl acetate (R. Langer, et al. Id.). This article is implanted at surgical sites from which tumors have been excised. Alternatively, the IL-4/TNF composition is encapsulated in semipermeable microcapsules or liposomes for injection into the tumor. This mode of administration is particularly useful for surgically inexcisable tumors, e.g. brain tumors.

The method for treating tumor cells by the administration of TNF and IL-4 to tumor bearing individuals contemplates both concurrent and sequential administration of these polypeptides. It will be known to one of skill in the art that the effect of concurrent administration will be mimicked by administering any one of the polypeptides to the individual followed by the subsequent administration of the second polypeptide. The timing of such sequential administration will be obvious to one of skill in the art and is dependent solely on the in vivo degradation rate of the first administered polypeptide.

The amount of the IL-4/TNF composition that is administered will depend, for example, upon the route of administration, the tumor in question and the condition of the patient. Intralesional injections will require less IL-4/TNF on a body weight basis than will intravenous infusion, while some tumor types, e.g. solid tumors, appear to be more resistant to tumor necrosis factor than others, e.g. leukemic. Accordingly, it will be necessary for the therapist to titer the dosage and modify the route of administration as required to obtain optimal cytotoxic activity towards the target tumor, as can be determined for example by biopsy of the tumor or diagnostic assays for putative cancer markers such as carcinoembryonic antigen, in view of any recombinant toxicity encountered at elevated dosage. Ordinarily, tumor necrosis factor dosages in mice (up to about 120 micrograms/kg body weight/day by intravenous administration) have been found to be substantially non-toxic and efficacious in vivo. IL-4 dosages in mice (up to about 120 micrograms/kg body weight/day by intravenous administration) have been found to be substantially non-toxic and efficacious in vivo.

Tumor necrosis factor is not believed to be species specific in its cytotoxic activity so that tumor necrosis factors other than human tumor necrosis factor, e.g. from bovine or porcine sources, might be employed in the present invention in therapy of human tumors. However, it is desired to use tumor necrosis factor from the species being treated in order to avoid the potential generation of autoantibodies.

Having now generally described the invention, a more complete understanding can be obtained by reference to the following specific examples.

### Example 1

### Cell Culture Procedures

All cell lines were strictly maintained in subconfluent cultures. Cells were plated at 0.3 - 1.3 x 10⁶ cells/25 mm petri dish in DMEM media supplemented with glutamine (2 mM), penicillin (100 units/ml), streptomycin (100 ug/ml) and fetal bovine serum (10 percent) in a humidifier incubator under 5 percent CO₂ in air.

### Example 2

### Cytotoxicity Assays

For cytotoxicity assays, 5 X 10³ cells in 0.1 ml of the medium were plated in 96 well costar plates. After overnight incubation at 37°C in a humidified atmosphere of 5 percent CO₂ in air, media was removed and a serial dilution of the test sample was layered in 0.1 ml of the medium. After 72 hours of incubation at 37°C, the media was removed and viable cells were monitored by crystal violet staining as described by Sugarman, et al. (1985) Science 230:943. The percentage of relative cell viability was calculated as the optical density in the presence of the test sample divided by the optical density in the absence of test sample (media) multiplied by 100.

### Example 3

### Radioreceptor Assay

Receptor binding assays were carried out as described Aggarwal, et al. (1985) Nature 318:665. Briefly, 0.25 X 10⁶ cells were incubated in 12 x 75 mm polypropylene tubes in 0.2 ml of fresh ice cold media (DMEM+10 percent FBS) containing 0.2 x 10⁶ cpm of ¹²⁵I-TNF either in the presence (nonspecific binding) or absence (total binding) of a 100-fold excess of unlabeled TNF. After 60 minutes incubation at 4°C, the media was removed, the cells were washed thrice by centrifugation, and cell bound radioactivity was determined. All determinations were made in triplicate.

### Example 4

### Enhancement of TNF Antiproliferative Effect by IL-4

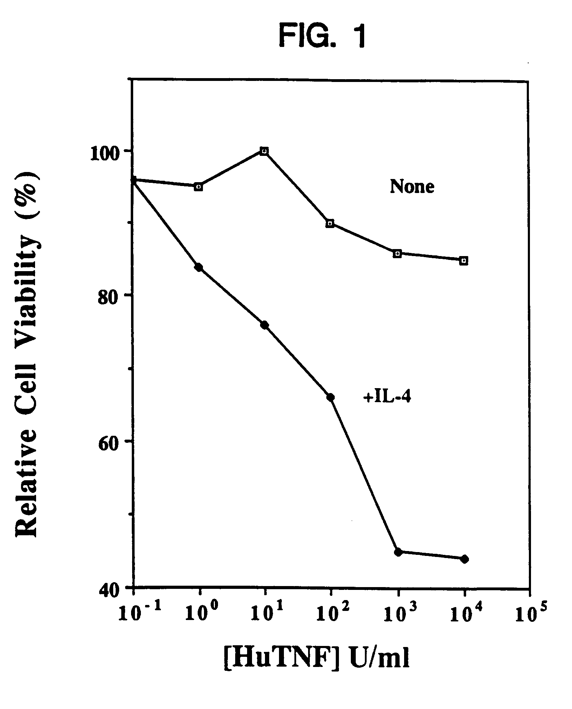

Interleukin-4 enhanced the antiproliferative effects of TNF when tested against different tumor cell types. Varying concentrations of TNF alone or in combination with a fixed concentration of IL-4 (100 ng/ml) were examined on a breast tumor cell line, MDA-MB 330. The results are shown in Figure 1. Following incubation of 5 x 10³ cells in 0.1 ml of the media containing the indicated amount of the cytokines for 72 hours, cell viability was determined as described in Example 1. All determinations were made in triplicate.

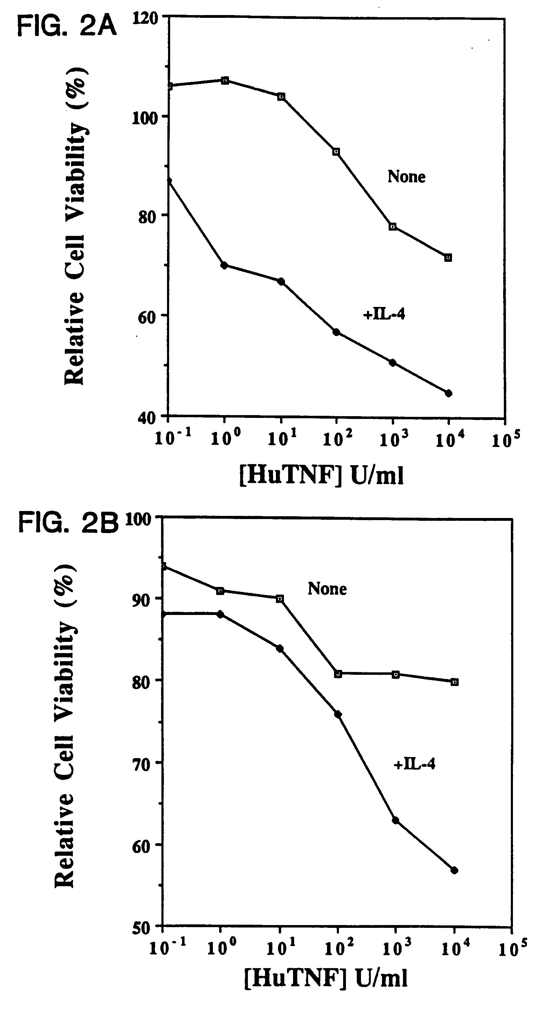

TNF alone, even at concentrations as high as 10,000 U/ml, had minimal effect on these cells. However, when IL-4 was added to the cultures with the TNF, the growth of these cells was inhibited in a dose dependent manner. As can be seen in Figure 1, only 10 percent cell growth inhibition was observed at concentration of 10,000 U/ml. However, when 100 ng/ml of IL-4 was also added, tumor cell growth inhibition was increased to almost 60 percent. The potentiation of cytotoxic effects of TNF by IL-4 was also observed with other cell types such as vulvar carcinoma cells (A431) and histiocytic lymphoma cells (U-937). Figures 2A and 2B demonstrate the effect of different concentrations of TNF and IL-4 (100 ng/ml) on the relative cell viability (percent) of human epithelial carcinoma cell line (A-431; Figure 2A) and human histiocytic lymphoma cell line (U-937; Figure 2B). 0.5 x 10³ cells were incubated in 0.1 ml of media containing the indicated amount of the cytokines for 72 hours, and thereafter the cell viability was determined as described in Example 1.

Figures 2A and 2B demonstrate that a significant effect of IL-4 on TNF-dependent growth modulation of A431 cells (Figure 2A) can be observed at concentrations as low as 0.01 U/ml of the cytokine.

The enhancement of cytotoxic effects of TNF by IL-4 was even more pronounced on U-937 cells (Figure 2B) at higher concentration (1000 U/ml).

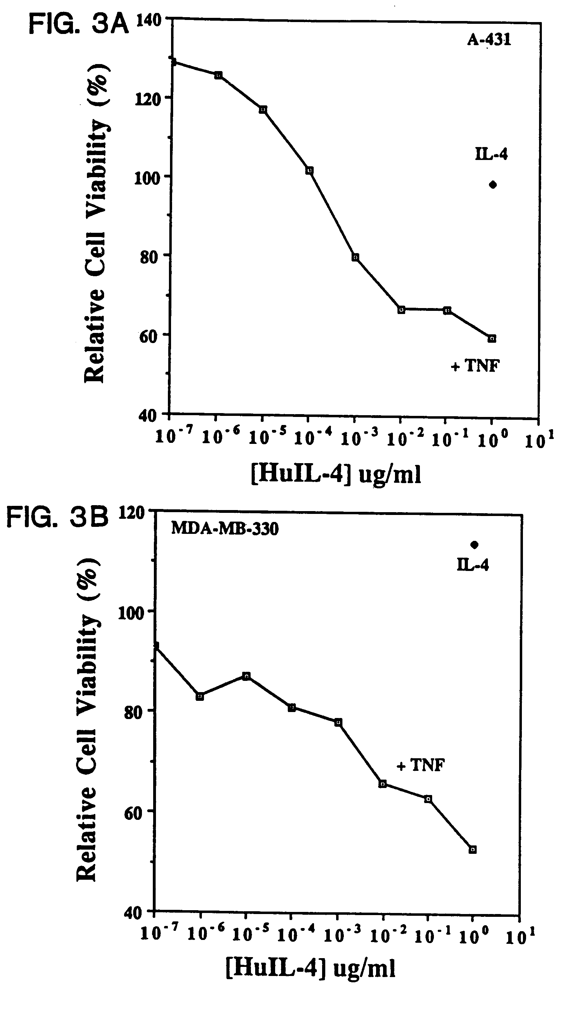

The effect of variable concentrations of IL-4 on a fixed concentration of TNF (1000 U/ml) was also investigated. 5 x 10³ cells were incubated with the indicated concentrations of the cytokines in 0.1 ml of the media for 72 hours, and thereafter the cell viability was determined as indicated in Example 1. All determinations were made in triplicate. Figures 3A and 3B demonstrate the effect of varying concentrations of IL-4 on the cytotoxicity of 1000 units/ml of TNF against A-431 (Figure 3A) and MDA-MB-330 (Figure 3B) cell lines. The effect of a single dose of IL-4 (1 ug/ml) alone is also shown. A-431 cells (Figure 3A) were inhibited in a dose dependent manner in the presence of both IL-4 and TNF. IL-4 alone was ineffective even at a concentration of 1 ug/ml. A similar dose dependent response of IL- 4 was observed with breast tumor cells (Figure 3B).

### Example 5

### Comparison of IL-4 and IFN-g Potentiation of TNF Cytotoxicity

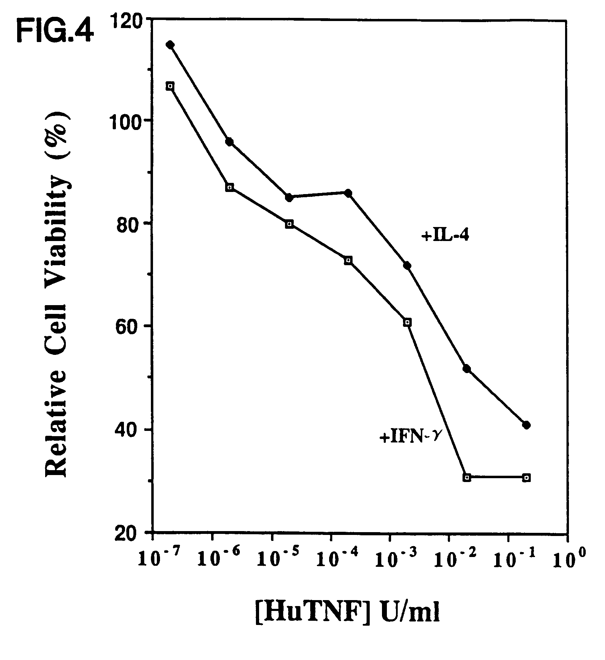

It has been previously demonstrated that interferon-g also enhances the cytotoxic effects of TNF against a wide variety of tumor cell lines ((U.S. Patent No. 4,650,674; Lee, et al. (1984) J. Immunol. 133:1003; Sugarman , et al. (1985) Science 230:943; Aggarwal, et al. (1985) Nature 318:665; Vilcek, et al. (1986) Interaction Between Tumor Necrosis Factor and Interferons. The Biology of the Interferon System, Edited by H. Schellekenes and W.E. Stewart. Elsevier Science Publishers, Amsterdam., page 249; Fransen, et al. (1986) Eur. J. Cancer Clin. Oncol. 22:419-426; Campbell, et al. (1988) J. Immunol. 141:2325-2329; Schiller, et al. (1987) Cancer Res. 47:2809-2813; Tsujimoto, et al. (1986) J. Immunol. 137:2272-2276). Figure 4 demonstrates the comparison of the effect of IL-4 and IFN-g on the TNF dependent cytotoxicity against MDA-MB-330 cells. 5 x 10³ cells were incubated in 0.1 ml of the media containing different concentrations of TNF and either IL-4 (lOO ng/ml) or IFN-g (lOO ng/ml) for 72 hours, and thereafter cell viability was determined as described in Example 1. All determinations were made in triplicate.

In order to determine whether IL-4 and IFN-g act by a similar mechanism to potentiate the cytotoxic effects of TNF, the potentiation of the antiproliferative response of TNF by IL-4 and interferon-g was compared. The results shown in Figure 4 indicate that on breast tumor cells both IL-4 and IFN-g at equal concentrations (lO0 ng/ml) enhance the growth inhibitory effects of TNF to a similar extent. Approximately 60-70 percent inhibition of cell growth was observed. Similar results were obtained with A-431 and U-937 cells (Table I).

**Table I**

| Effect of Interleukin-4 and Interferon Gamma on the TNF Dependent Antiproliferative Effects Against Various Human Tumor Cell Line¹ | | | |
|---|---|---|---|
| Cytokine | Relative Cell Viability (Percent) | | |
| | MDA-MB-330 | A-431 | U-937 |
| None | 100 | 100 | l00 |
| TNF | 80 | 71 | 89 |
| IL-4 | 103 | 86 | 104 |
| IFN-g | 82 | 69 | 104 |
| TNF+IFN-g | 42 | 33 | 50 |
| TNF+IL-4 | 51 | 45 | 64 |
| IFN-g + IL-4 | 60 | 48 | 89 |
| TNF+IFN-g+IL-4 | 38 | 20 | 41 |

| | | | |
|---|---|---|---|
| ¹ 5x10³ cells were incubated in 0.1 ml volume of media containing either TNF (200 ng/ml) or IL-4 (100 ng/ml) or IFN-g (100 ng/ml) or combination of cytokines as indicated above. In case of A-431 cells, concentration of all cytokines were the same except IFN-g (1 ng/ml). After 72 hours incubation with cytokines, the cell viability was determined by crystal violet staining as indicated in Example 1. | | | |

IL-4 also appears to slightly enhance the antiproliferative effects of IFN-g, although not to the same extent as TNF (Table I). Furthermore, the combination of all three cytokines together was maximally effective. For instance, when tested on A431 cells, only 30 percent inhibition of cell growth was observed with TNF, 55 percent when IL-4 and TNF were added together, and 80 percent when all three cytokines were present in combination, i.e. IFN-g, IL-4 and TNF are present (Table I).

These results indicate that the mechanism by which IFN-g enhances the cytotoxic response of TNF is different from that of IL-4. In order to further demonstrate the difference in mechanism of potentiation of TNF by IL-4 and IFN-g, the effect of both cytokines on the induction of the TNF receptor was determined. IFN-g is known to induce the receptor for TNF. The results shown in Table II clearly indicate that preexposure of U-937 cells to IL-4 overnight does not cause an increase in the specific binding of TNF, suggesting no increase in synthesis of new receptors. Therefore, the mechanism of IL-4 potentiation of TNF is different from that of IFN-g.

**Table II**

| Effect of Interleukin-4 on the Binding of Tumor Necrosis Factor to U.937 cells¹ | | | |
|---|---|---|---|
| Treatment | Total Binding | Non-specific Binding | Specific Binding |
| | (Counts Per Minute) | | |
| None | 1271+20 | 264+43 | 1007 |
| Interleukin-4 | 1203+50 | 277+25 | 926 |

| | | | |
|---|---|---|---|
| ¹ U 937 cells were treated with IL-4 (1 ug/ml) for 24 hours at 37°C and then washed with the media. 250,000 cells were incubated at 4°C for one hour in 0.2ml of media containing 0.2 x 10⁶ cpm of ^{I25}I-TNF in the presence or absence of l00 nm of unlabeled TNF. Thereafter, cells were washed and cell bound radioactivity was determined as described in Example 2. All determinations were made in triplicate. | | | |

IL-4 when added alone caused proliferation of certain cells. As shown in Table III, IL-4 enhanced the growth of the breast tumor cell line ZR-75-1 by almost 40 percent over the control. Cell proliferative effects were also observed with some of the other cell lines but to a lesser degree. The growth of A-431 cells was slightly inhibited (Table II) by IL-4. These growth stimulatory effects of IL-4 were found to be dose dependent.

**Table III**

| Modulation of Growth of Various Human Tumor Cell Lines by Interleukin-4¹ | |
|---|---|
| Cell Types | Relative Cell Viability (Percent) |
| ZR-75-l(Breast tumor) | 140 |
| MDA-MB-4GS(Breast tumor) | 130 |
| HS-OS7ST(Breast tumor) | 132 |
| BT-20(Breast tumor) | 110 |
| MCF-7 (Breast tumor) | 125 |
| A-375(Melanoma) | 120 |
| MDA-MB-330(Breast tumor) | 110 |
| A-431(Vulvar Carcinoma) | 86 |
| U-937 (Histiocytic lymphoma) | 110 |

| | |
|---|---|
| ¹ 5X10³ cells were incubated with Interleukin-4 (l ug/ml) for 72 hours, and then the relative cell viability was determined by crystal violet staining according to Example 1. All determinations were made in triplicate. | |

### Example 6

### Enhancement of Lymphotoxin Antiproliferative Effect by IL-4

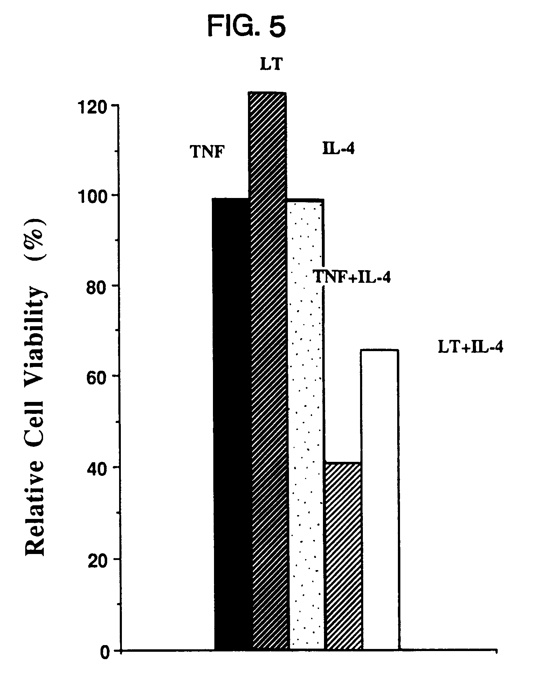

Interleukin-4 enhanced the antiproliferative effects of Lymphotoxin (Lt) when tested against tumor cells. The antiproliferative effect of Lt (1000 U/ml) alone or in combination with IL-4 (1 ug/ml) was compared with the effect of TNF (1000 U/ml) alone or in combination with IL-4 (1 ug/ml) on a breast tumor cell line, MDA-MB 330. Following incubation of 5 x 10³ cells in 0.1 ml of the media containing the indicated amount of the cytokines for 72 hours, cell viability was determined as described in Example 1. All determinations were made in triplicate. The results are shown in Figure 5.

TNF alone had minimal effect on these cells, inhibiting cell growth by only 5 percent. Lt alone had a slightly proliferative effect on these cells. The cell viability in the presence of Il-4 alone was 95-98 percent. However, when IL-4 was added to the cultures with the TNF or Lt, the growth of these cells was significantly inhibited. As can be seen in Figure 5, tumor cell growth inhibition was increased to almost 60 percent in the presence of IL-4 and TNF, and 40 percent in the presence of IL-4 and LT.

The mechanism by which IFN-g potentiates TNF response is not understood. It is known that upregulation of cytotoxic effects of TNF by IFN-g accompanies the upregulation of TNF receptors (Aggarwal, et al. (1985) Nature 318:665; Vilcek, et al. (1986) The Biology of the Interferon System, page 249). Our studies, however, show that IL-4 does not upregulate TNF receptors on cells which are affected by it. Even though these observations imply that the mechanism of enhancement of TNF response by IL-4 is different from that of IFN-g, it has been reported that upregulation of TNF receptors by IFN-g is not sufficient either for their synergistic cytotoxic response (Tsujimoto, et al. (1986) J. Immunol. 137:2272-2276; Aggarwal, et al. (1987) J. Biol. Chem. 262:10000-10007). Our observations also indicate that IFN-g potentiates the response of TNF and IL-4 together. Therefore, it is possible that these two agents are working through independent mechanisms. Interleukin-1 is also known to enhance the cytotoxic effects of TNF (Ruggiero and Baglioni (1987) J. Immunol. 138:0661-0663; Holtmann and Wallach (1987) J. Immunol. 139:1161-1167). Thus, the mechanism by which IL-4 potentiates the effects of TNF is also not clear. In contrast to IFN-g, IL-1 has been shown to downregulate the receptors for TNF. Besides cytokines, other agents such as inhibitors of protein synthesis and elevated temperature can increase the TNF dependent cytotoxicity (Watanabe, et al. (1988) Cancer Res. 48:650-653; Niitsu, et al. (1988) Cancer Res. 48:654-657). Studies with protein synthesis inhibitors imply that TNF induces the synthesis of certain proteins which protects the cells from the cytotoxic effect of TNF (Himeno, et al. (1990) Cancer Res. 50:4941- 4945; Watanabe, et al. (1988) Immunopharmacol. Immunotoxicol. 10:479-499). Both epidermal growth factor and transforming growth factor are known to inhibit the antiproliferative effects of TNF (Sugarman, et al. (1987) Cancer Res. 47:780-786) against several cell types. These growth factors are known to be produced by various tumor cells. Therefore, it is possible that IL-4 functions by inhibiting the synthesis of these growth factors. It is also possible that IL-4 functions by downregulating the expression of c-fos and c-myc oncogenes, which are known to be involved in cell proliferation.

The cDNA's for receptors of TNF, IFN-g and IL-4 have been cloned recently (Idzerda, et al. (1990) J. Exp. Med. 171:861-873; Schall, et al. (1990) Cell 61:361-370; Loetscher, et al. (1990) Cell 61:351-359; Smith, et al. (1990) Science 248:1019-1023; Auget, et al. (1988) Cell 55:273-280). The structure of these receptors, however, have revealed that the receptor by itself is not sufficient for signal transduction. It is known that IL-4 can downmodulate the production of TNF from macrophages (Essner, et al. (1989) J. Immunol. 142:3857; Hamilton, (1989) Proc. Natl. Acad. Sci. USA 86:3803), but it has also been reported that IL-4 can enhance macrophage mediated cytotoxicity (Somers and Erickson (1989) Cell Immunol. 122:178-187). The latter has been shown to be primarily due to TNF (Feinman, et al. (1987) J. Immunol. 138:635-640). Therefore, it is not clear how inhibition of TNF production accompanies with the enhancement of macrophage cytotoxicity by IL-4. It is possible that enhancement of macrophage cytotoxicity toward tumor cells by IL-4 is mediated by the synergistic effects of TNF and IL-4 together described herein. It has been shown that IL-4 producing tumor cells exhibit antitumor activity in vivo against other tumors (Tepper, et al. (1989) Cell 57:503-512). Since IL-4 alone does not directly produce an antiproliferative effect in tumor cells in culture, the mechanism by which IL-4 acts as an antitumor agent in vivo is not understood. It is possible that in this situation IL-4 acts as an antitumor agent by both activating macrophages as well as by potentiating the antitumor effects of TNF.

## Claims

1. The use of a cell-free composition comprising Interleukin-4 and Tumour Necrosis Factor in a unit activity proportion of from 0.1:1 to 200:1 for the manufacture of a medicament for the treatment of tumour cells.

2. The use according to claim 1, wherein the Tumour Necrosis Factor is human Tumour Necrosis Factor.

3. The use according to either claim 1 or claim 2, wherein the Interleukin-4 is human Interleukin-4.

4. The use according to any one of claims 1 to 3, wherein the composition comprises Interleukin-4 and Tumour Necrosis Factor in a unit activity proportion of 10:1.

5. The use of a cell-free composition comprising Interleukin-4 and lymphotoxin in a unit activity proportion of from 0.1:1 to 200:1 for the manufacture of a medicament for the treatment of tumour cells.

6. The use according to claim 5, wherein the Interleukin-4 is human Interleukin-4.

7. The use according to either claim 5 or claim 6, wherein the composition additionally comprises interferon.

## Patentansprüche

1. Verwendung einer zellfreien Zusammensetzung umfassend Interleukin-4 und Tumor-Nekrose-Faktor in einem Einheitsaktivität-Verhältnis von 0,1:1 bis 200:1 zur Herstellung eines Arzneimittels zur Behandlung von Tumorzellen.

2. Verwendung nach Anspruch 1, dadurch gekennzeichnet, daß der Tumor-Nekrose-Faktor menschlicher Tumor-Nekrose-Faktor ist.

3. Verwendung nach Anspruch 1 oder Anspruch 2, dadurch gekennzeichnet, daß das Interleukin-4 menschliches Interleukin-4 ist.

4. Verwendung nach einem der Ansprüche 1 bis 3, dadurch gekennzeichnet, daß die Zusammensetzung Interleukin-4 und Tumor-Nekrose-Faktor in einem Einheitsaktivität-Verhältnis von 10:1 umfaßt.

5. Verwendung einer zellfreien Zusammensetzung umfassend Interleukin-4 und Lymphotoxin in einem Einheitsaktivität-Verhältnis von 0,1:1 bis 200:1 zur Herstellung eines Arzneimittels zur Behandlung von Tumorzellen.

6. Verwendung nach Anspruch 5, dadurch gekennzeichnet, daß das Interleukin-4 menschliches Interleukin-4 ist.

7. Verwendung nach Anspruch 5 oder Anspruch 6, dadurch gekennzeichnet, daß die Zusammensetzung zusätzlich Interferon umfaßt.

## Revendications

1. Utilisation d'une composition exemple de cellules comprenant une interleukine-4 et un facteur de nécrose tumorale en une proportion exprimée en unités d'activité de 0,1 : 1 à 200 : 1 pour la fabrication d'un médicament pour le traitement des cellules tumorales.

2. Utilisation selon la revendication 1, dans laquelle le facteur de nécrose tumorale est le facteur de nécrose tumorale humain.

3. Utilisation selon la revendication 1 ou la revendication 2, dans laquelle l'interleukine-4 est de l'interleukine-4 humaine.

4. Utilisation selon une quelconque des revendications 1 à 3, dans laquelle la composition comprend une interleukine-4 et un facteur de nécrose tumorale en une proportion exprimée en unités d'activité de 10 : 1.

5. Utilisation d'une composition exempte de cellules comprenant une interleukine-4 et une lymphotoxine en une proportion exprimée en unités d'activité de 0,1 : 1 à 200 : 1 pour la fabrication d'un médicament pour le traitement des cellules tumorales.

6. Utilisation selon la revendication 5, dans laquelle l'interleukine-4 est de l'interleukine-4 humaine.

7. Utilisation selon la revendication 5 ou la revendication 6, dans laquelle la composition contient en outre un interféron.
